(19) Europäisches Patentamt
European Patent Office
Office européen des brevets

(11) Veröffentlichungsnummer: **0 591 765 A2**

(12) # EUROPÄISCHE PATENTANMELDUNG

(21) Anmeldenummer: **93115262.3**

(51) Int. Cl.[5]: **C07D 211/90**, A61K 31/445

(22) Anmeldetag: **22.09.93**

(30) Priorität: **05.10.92 DE 4233347**

(43) Veröffentlichungstag der Anmeldung: **13.04.94 Patentblatt 94/15**

(84) Benannte Vertragsstaaten: **AT BE CH DE DK ES FR GB GR IE IT LI LU MC NL PT SE**

(71) Anmelder: **BAYER AG**
**D-51368 Leverkusen(DE)**

(72) Erfinder: **Hartwig, Wolfgang, Dr.**
**Am Rohm 109**
**D-42113 Wuppertal(DE)**
Erfinder: **Niewöhner, Ulrich, Dr.**
**Gartenstrasse 3**
**D-42929 Wermelskirchen(DE)**
Erfinder: **Perzborn, Elisabeth, Dr.**
**Am Tescher Busch 13**
**D-42327 Wuppertal(DE)**
Erfinder: **Schramm, Matthias, Dr.**
**Humperdinckstrasse 15**
**D-51375 Leverkusen(DE)**
Erfinder: **Knorr, Andreas, Dr.**
**Trillser Graben 10**
**D-40699 Erkrath(DE)**

(54) **1,4-Dihydropyridin-alkoxy- und-alkylamino-ester als antihypertensiva.**

(57) Dihydropyridin-alkoxy- und -alkylamino-aroylester werden durch Umsetzung von Salicylsäure mit entsprechenden Aminoalkylester- oder Hydroxyalkylester-dihydropyridinen hergestellt. Die Dihydropyridin-alkoxy- und -alkylamino-aroylester eignen sich als Wirkstoffe in Arzneimitteln, insbesondere zur Behandlung von Herz-, Kreislauf-, thromboembolischen sowie cerebralen Erkrankungen.

EP 0 591 765 A2

Die Erfindung betrifft Dihydropyridin-alkoxy- und -alkylamino-aroylester, Verfahren zu ihrer Herstellung, sowie ihre Verwendung in Arzneimitteln, insbesondere für Herz-, Kreislauf und thromboembolische Erkrankungen und als cerebral wirksame Mittel.

Die vorliegende Erfindung betrifft Dihydropyridin-alkoxy- und -alkylaminoaroylester der allgemeinen Formel (I)

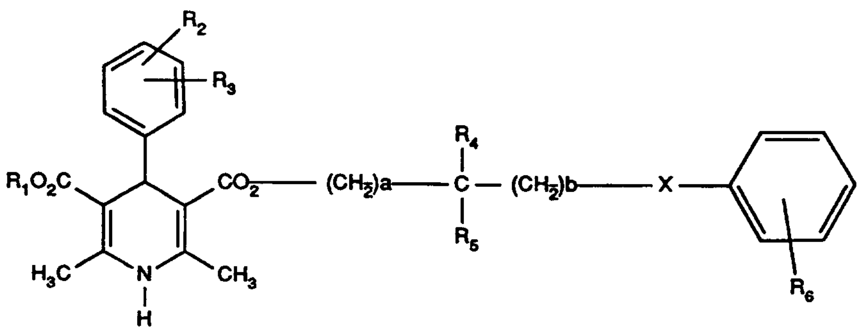

(I),

in welcher

$R^1$ für geradkettiges oder verzweigtes Alkyl mit bis zu 8 Kohlenstoffatomen steht, das gegebenenfalls durch geradkettiges oder verzweigtes Alkoxy mit bis zu 6 Kohlenstoffatomen substituiert ist, oder
für Cycloalkyl mit 3 bis 8 Kohlenstoffatomen steht,

$R^2$ für Halogen, Trifluormethyl, Nitro oder Cyano steht,

$R^3$ die oben angegebene Bedeutung von $R^2$ hat und mit dieser gleich oder verschieden ist oder für Wasserstoff steht,

a und b gleich oder verschieden sind und für eine Zahl 0, 1, 2, 3 oder 4 stehen,

$R^4$ und $R^5$ gleich oder verschieden sind und für Wasserstoff, geradkettiges oder verzweigtes Alkyl mit bis zu 6 Kohlenstoffatomen oder Phenyl stehen,

X für eine Gruppe der Formel -O-CO- oder -NH-CO- steht,

$R^6$ für Hydroxy oder für den Rest der Formel

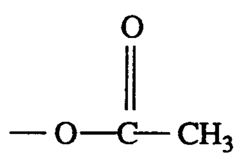

steht

und deren Salze.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Als Salze der erfindungsgemäßen Verbindungen sind physiologisch unbedenkliche Salze bevorzugt.

Physiologisch unbedenkliche Salze sind Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Bevorzugt sind Verbindungen der allgemeinen Formel (I),
in welcher

$R^1$ für geradkettiges oder verzweigtes Alkyl mit bis zu 6 Kohlenstoffatomen steht, das gegebenenfalls durch geradkettiges oder verzweigtes Alkoxy mit bis zu 4 Kohlenstoffatomen substituiert ist, oder
für Cyclopropyl, Cyclopentyl oder Cyclohexyl steht,

$R^2$ für Fluor, Chlor, Brom, Trifluormethyl, Nitro oder Cyano steht,

$R^3$ die oben angegebene Bedeutung von $R^2$ hat und mit dieser gleich oder verschieden ist, oder für Wasserstoff steht,

a und b gleich oder verschieden sind und für eine Zahl 0, 1, 2 oder 3 stehen,

$R^4$ und $R^5$ gleich oder verschieden sind und für Wasserstoff, geradkettiges oder verzweigtes Alkyl mit bis zu 4 Kohlenstoffatomen oder Phenyl stehen,

X für eine Gruppe der Formel -O-CO- oder -NH-CO- steht,

$R^6$ für Hydroxy oder für den Rest der Formel

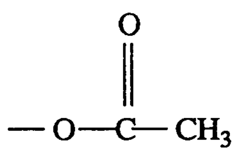

steht

und deren Salze.

Besonders bevorzugt sind Verbindungen der allgemeinen Formel (I),
in welcher

$R^1$ für geradkettiges oder verzweigtes Alkyl mit bis zu 4 Kohlenstoffatomen steht, das gegebenenfalls durch Methoxy substituiert ist, oder
für Cyclopentyl oder Cyclohexyl steht,

$R^2$ für Fluor, Chlor, Trifluormethyl, Nitro oder Cyano steht,

$R^3$ die oben angegebene Bedeutung von $R^2$ hat und mit dieser gleich oder verschieden ist oder für Wasserstoff steht,

a und b gleich oder verschieden sind und für eine Zahl 0, 1 oder 2 stehen,

$R^4$ und $R^5$ gleich oder verschieden sind und für Wasserstoff, Methyl oder Phenyl stehen,

X für eine Gruppe der Formel -O-CO oder -NH-CO- steht,

$R^6$ für Hydroxy oder für den Rest der Formel

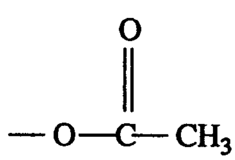

steht,

und deren Salze.

Ganz besonders bevorzugt sind
5-[2-(2-Acetoxybenzoyloxy)ethoxycarbonyl]-2,6-dimethyl-4-(2-trifluormethylphenyl)-1,4-dihydropyridin-3-carbonsäuremethylester,
5-[2-(2-Acetoxybenzoyloxy)ethoxycarbonyl]-2,6-dimethyl-4-(2-trifluormethylphenyl)-1,4-dihydropyridin-3-carbonsäureethylester,
5-[2-(2-Acetoxybenzoyloxy)ethoxycarbonyl]-2,6-dimethyl-4-(2,3-dichlorphenyl)-1,4-dihydropyridin-3-carbonsäuremethylester.

Außerdem wurden Verfahren zur Herstellung der erfindungsgemäßen Verbindungen der allgemeinen Formel (I) gefunden, dadurch gekennzeichnet, daß man

[A] im Fall, daß X für die -NH-CO-Gruppe steht, Aminoverbindungen der allgemeinen Formel (II)

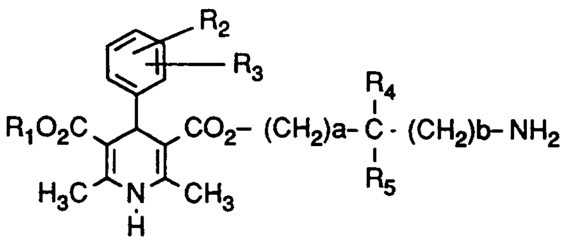

(II),

in welcher
$R^1$, $R^2$, $R^3$, $R^4$, $R^5$, a und b die oben angegebene Bedeutung haben,
mit Salicylsäure der allgemeinen Formel (III)

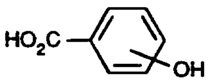

(III)

in inerten Lösemitteln, gegebenenfalls in Anwesenheit einer Base und/oder Hilfsstoffes umsetzt,
im Fall, daß $R^6$ für die $-O-CO-CH_3$-Gruppe steht, mit Acetanhydrid oder Acetylchlorid in Anwesenheit einer Base und eines Hilfsstoffes acetyliert,
[B] und im Fall, daß X für die -O-CO-Gruppe steht,

Verbindungen der allgemeinen Formel (IV)

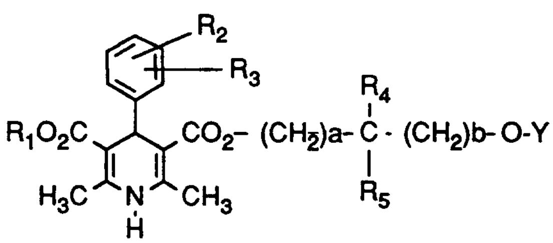

(IV),

in welcher
$R^1$, $R^2$, $R^3$, $R^4$, $R^5$, a und b die oben angegebene Bedeutung haben
und
Y für eine aktivierende Gruppe wie beispielsweise $-SO_3CH_3$, $-SO_3-(C_6H_5)-p-CH_3$ steht,
nach üblichen Methoden einer alkylierenden Veresterung
mit Acetylsalicylsäure der Formel (V)

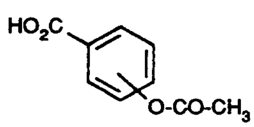

(V)

in inerten Lösemitteln, in Anwesenheit einer Base umsetzt,
und im Fall, daß $R^6$ für die Hydroxyschutzgruppe steht, die Acetylgruppe, ebenfalls nach bekannten Methoden, abspaltet.

Die erfindungsgemäßen Verfahren können durch folgendes Formelschema beispielhaft erläutert werden:

[A]

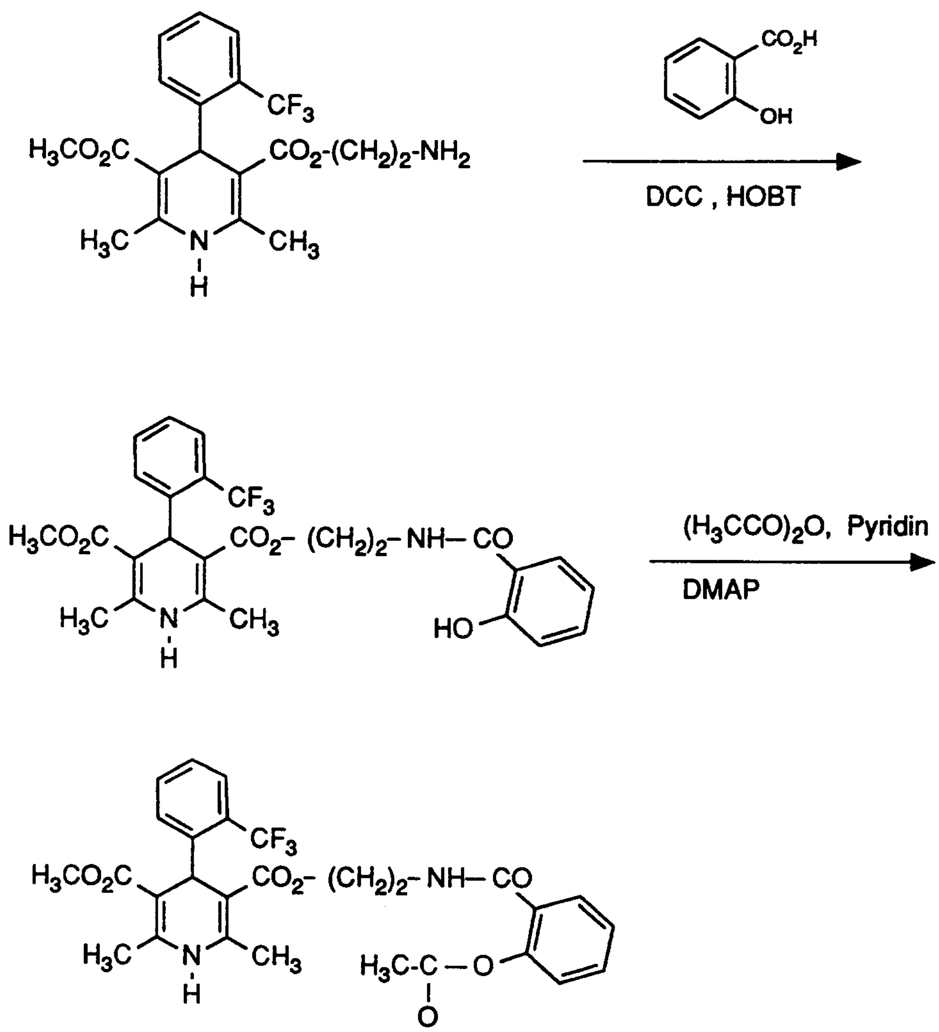

[B]

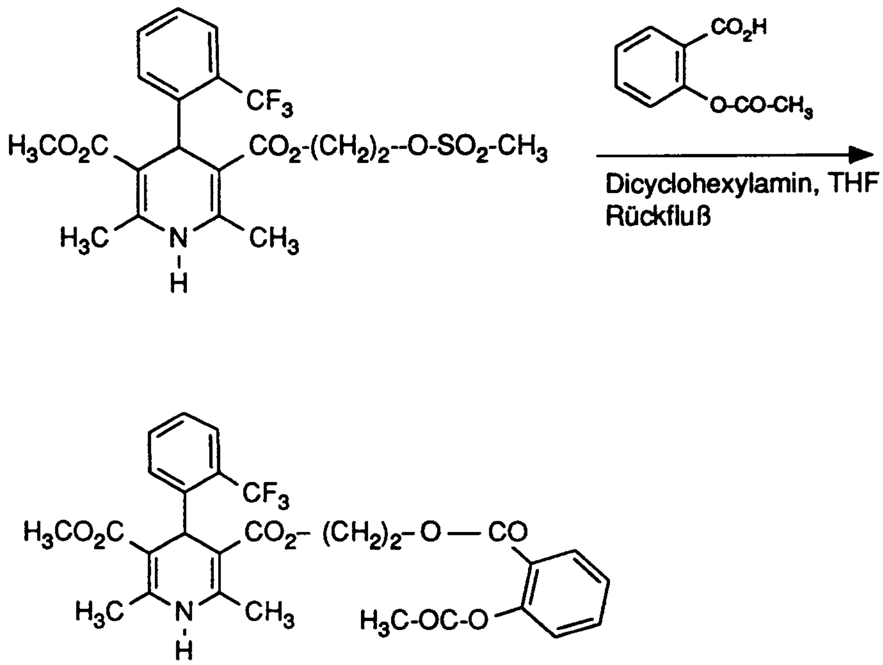


Lösemittel für die erfindungsgemäßen Verfahren [A] und [B] können hier inerte organische Lösemittel sein, die sich unter den Reaktionsbedingungen nicht verändern. Hierzu gehören bevorzugt Ether wie beispielsweise Diethylether, Dioxan, Tetrahydrofuran, Glykolmono- oder dimethylether, Halogenkohlenwasserstoffe wie Di-, Tri- oder Tetrachlormethan, Dichlorethylen, Trichlorethylen, Essigester, Toluol, Acetonitril, Pyridin, Hexamethylphosphorsäuretriamid und Aceton. Selbstverständlich ist es möglich, Gemische der Lösemittel einzusetzen.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Die Reaktionstemperatur für die Verfahren [A] und [B] können in einem größeren Bereich variiert werden. Im allgemeinen arbeitet man in einem Bereich von - 20 °C bis 200 °C, bevorzugt von 0 °C bis 25 °C.

Die Verfahren können bei Normaldruck, erhöhtem oder erniedrigtem Druck (beispielsweise von 0,5 bis 5 bar), vorzugsweise bei Normaldruck, durchgeführt werden.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Als Basen eignen sich im allgemeinen Alkalicarbonate wie beispielsweise Natrium-oder Kaliumcarbonat, oder organische Basen wie Trialkylamine, z.B. Triethylamin, N-Ethylmorpholin, N-Methylpiperidin oder N-Ethyl-diisopropylamin, oder Dimethylaminopyridin (DMAP), 1,8-Diazabicyclo[5.4.0]undec-7-en (DBU), 1,5-Diazabicyclo[4.3.0]non-5-en (DBN) oder Dicyclohexylamin.

Die Base wird im allgemeinen in einer Menge von 0,01 mol bis 1 mol, bevorzugt von 0,05 mol bis 0,1 mol, jeweils bezogen auf 1 mol der Verbindungen der allgemeinen Formeln (II) oder (IV), eingesetzt.

Enantiomerenreine Formen erhält man z.B., indem man Diastereomerengemische der Verbindungen der allgemeinen Formel (VI)

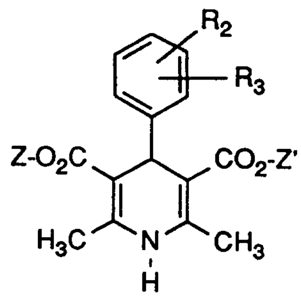

(VI),

in welcher

$R^2$ und $R^3$ die oben angegebene Bedeutung haben

und

Z und Z' für einen optischen Esterrest stehen,

nach üblicher Methode trennt, anschließend die enantiomerenreinen Carbonsäuren herstellt und dann gegebenenfalls durch Veresterung mit entsprechenden Alkoholen in die enantiomerenreinen Dihydropyridine überführt.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Bevorzugt erfolgt die Trennung der Enantiomere der Verbindungen der allgemeinen Formel (VI) mit Z/Z' = H durch Säulenchromatographie nach üblicher Methode, wobei die Säulen mit chiralen Trägermaterialien gefüllt sind.

Die Acetylierung erfolgt im allgemeinen, ausgehend von der entsprechenden Hydroxyverbindung, in einem der oben angegebenen Lösemitteln, vorzugsweise in Pyridin, mit Acetanhydrid, in Anwesenheit einer der oben aufgeführten Basen, vorzugsweise Dimethylaminopyridin, bei Raumtemperatur und Normaldruck.

Die Verbindungen der allgemeinen Formel (VI) sind teilweise bekannt oder können dann nach üblichen Methoden hergestellt werden.

Die vorstehenden Herstellungsverfahren sind lediglich zur Verdeutlichung angegeben. Die Herstellung der Verbindungen der Formel (I) sind nicht auf diese Verfahren beschränkt sondern jede Modifikation dieser Verfahren ist in gleicher Weise für die Herstellung der erfindungsgemäßen Verbindungen anwendbar.

Die Verbindungen der allgemeinen Formeln (II) und (IV) sind größtenteils bekannt [vgl. US 44 19 518] oder können nach den in der Dihydropyridinchemie üblichen Methoden, beispielsweise unter Aktivierung der jeweiligen Carbonsäuren mit einem der oben aufgeführten Hilfsstoffe, vorzugsweise Dicyclohexylcarbodiimid oder Carbonyldiimidazol und nachfolgender Umsetzung mit Verbindungen der allgemeinen Formel (VII) und (VIIa)

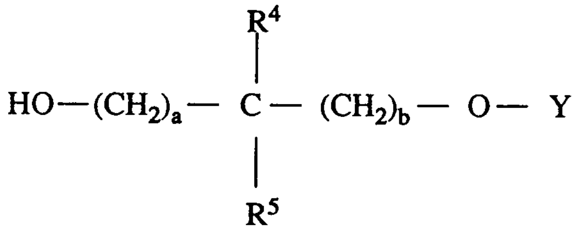

(VII)

,

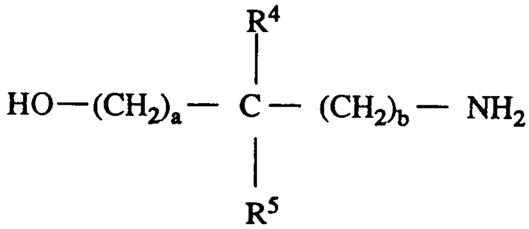

(VIIa)

in welcher

a, b, $R^4$, $R^5$ und Y die oben angegebene Bedeutung haben hergestellt werden.

Die Verbindungen der allgemeinen Formeln (VII) und (VIIa) sind käuflich oder können nach üblichen Methoden hergestellt werden.

Die Salicylsäure (III) und Acetylsalicylsäure (V) sind bekannt.

Die erfindungsgemäßen Verbindungen der allgemeinen Formel (I) zeigen ein nicht vorhersehbares, wertvolles pharmakologisches Wirkspektrum. Sie beeinflussen zum einen die Kontraktionskraft des Herzens, den Tonus der glatten Muskulatur sowie den Elektrolyt- und Flüssigkeitshaushalt. Außerdem weisen sie eine thrombozytenaggregationshemmende Wirkung auf. Sie können daher in Arzneimitteln zur Behandlung des pathologisch veränderten Blutdrucks und der Herzinsuffizienz, sowie als Koronartherapeutika eingesetzt werden. Sie können zur Behandlung von thrombotischen Erkankungen und Ischämien wie Myokardinfarkt, Hirnschlag, transistorische und ischämische Attacken, Angina pectoris, periphere Durchblutungsstörungen, Verhinderung von Okklusion und Restenosen wie nach Thrombolysetherapie, percutan transluminalen Angioplastien (PTA), percutanen transluminalen Koronarangioplastien (PTCA), Bypass sowie zur Behandlung von Arteriosklerose und cerebralen Erkrankungen eingesetzt werden.

Die Substanzen wurden an der wachen Ratte auf ihre blutdrucksenkende Wirkung hin untersucht. An wachen Ratten mit genetisch bedingtem Hochdruck ("spontan hypertone Ratten" des Okamoto-Stammes) wird der arterielle Blutdruck mit der "Schwanz-Manchette" in definierten Zeitabständen nach Substanzgabe unblutig gemessen. Die zu prüfenden Substanzen werden aufgeschwemmt in einer Tylosesuspension intragastral ("oral") per Schlundsonde in verschiedenen Dosen appliziert. Die erfindungsgemäßen Verbindungen senken den arteriellen Blutdruck der Hochdruckratten in klinisch relevanter Dosierung.

| Es ergaben sich folgende Wirkungen: | |
|---|---|
| SH-Ratte: Blutdrucksenkung >15 mm | |
| Bsp.-Nr. | Grenzdosis [mg/kg] |
| 1 | 10 |
| 2 | 30 |
| 3 | 10 |
| 4 | 30 |
| 5 | 10 |
| 6 | 3 |

Bei diesen Ratten wurde auch die Thrombozytenaggregationshemmung ex vivo (Ratte) nach folgender Methode untersucht.

Die Prüfsubstanzen (10 mg/kg) bzw. Lösemittel werden mittels Schlundsonde Ratten appliziert. Nach 120 Minuten wird den Tieren unter Ethernarkose über die Bauchaorta Blut entnommen. Das Blut wird in 3,8%iger Citrat-Lösung (9 +1) aufgenommen. Anschließend wird durch Zentrifugation bei 150 g für 20

Minuten plättchenreiches Plasma (PRP) gewonnen. 3 Teile PRP werden mit 1 Teil 0,9% NaCl verdünnt und bei 37 °C 5 Minuten vorinkubiert.

Die Plättchenaggregation wird nach der turbidometrischen Methode [vgl. Born, G.V.R.; J. Physiol 162, 67, 1962] im Aggregometer bei 37 °C bestimmt. Hierzu wird PRP mit Kollagen, einem aggregationsauslösenden Agens, versetzt. Die Prüfung erfolgt jeweils bei Schwellenkonzentrationen von Kollagen, die beim Kontrollansatz maximale Aggregation bewirken. Die Veränderung der optischen Dichte der aggregierenden Probe wird erfaßt und der maximale Ausschlag bestimmt. Hierzu wird die prozentuale Hemmung gegenüber der Kontrolle errechnet.

Es ergab sich eine Hemmung von mehr als 50%.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,01 bis 10 mg/kg, vorzugsweise etwa 0,1 bis 3 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

5-[2-(2-Acetoxybenzoyloxy)ethoxycarbonyl]-2,6-dimethyl-4-(2-trifluormethylphenyl)-1,4-dihydropyridin-3-carbonsäuremethylester

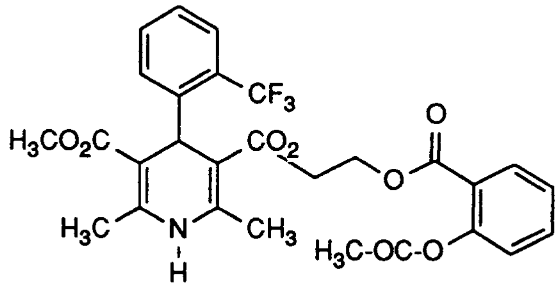

3,85 g (8 mmol) 5-[2-Mesyloxyethoxycarbonyl)-2,6-dimethyl-4-(2-trifluormethylphenyl)-1,4-dihydropyridin-3-carbonsäuremethylester, 1,5 g (8,3 mmol) Acetylsalicylsäure und 1,5 ml N-Ethyl-diisopropylamin werden 4 h in 60 ml THF refluxiert. Es wird eingedampft, der Rückstand in 50 ml Essigsäureethylester aufgenommen und mit 2 n HCl-Lösung, dann mit gesättigter $NaHCO_3$-Lösung und Wasser ausgeschüttelt. Nach Trocknen über $Na_2SO_4$ wird die organische Phase eingedampft und der Rückstand durch Chromatographie über Kieselgel (Eluens: Toluol / Essigsäureethylester 10:1) gereinigt.

Ausbeute: 2,78 g (62%) farbloses Öl
$R_f$ = 0,49 (Toluol/Aceton 4:1)
In Analogie zur Vorschrift des Beispiels 1 werden die in Tabelle 1 aufgeführten Verbindungen hergestellt:

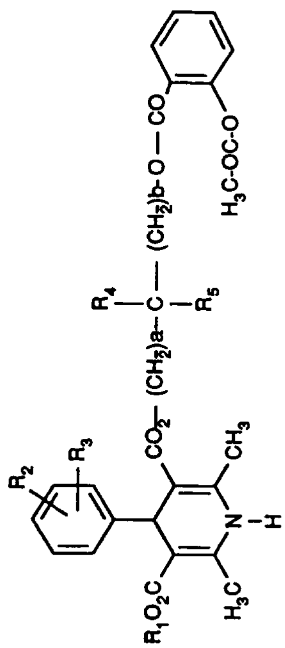

Tabelle 1:

| Bsp.-Nr. | $R^1$ | $R^2$ | $R^3$ | $R^4$ | $R^5$ | a | b | Ausbeute (% d.Th.) | $R_f$ (Toluol/Aceton 4:1) |
|---|---|---|---|---|---|---|---|---|---|
| 2 | $-CH(CH_3)_2$ | $2-NO_2$ | H | H | H | 1 | 0 | 65 | 0,42 |
| 3 | $-CH(CH_3)_2$ | $2-CF_3$ | H | H | H | 1 | 0 | 51 | 0,46 |
| 4 | $-CH(CH_3)_2$ | 2-Cl | H | H | H | 1 | 0 | 57 | 0,51 |
| 5 | $-C_2H_5$ | $2-CF_3$ | H | H | H | 1 | 0 | 44 | 0,45 |
| 6 | $-CH_3$ | 2-Cl | 3-Cl | H | H | 1 | 0 | 62 | 0,50 |
| 7 | $-CH_3$ | $2-CF_3$ | H | H | H | 2 | 1 | 31 | 0,44 |
| 8 | $-CH_3$ | $2-CF_3$ | H | $-CH_3$ | $-CH_3$ | 1 | 1 | 41 | 0,48 |
| 9 | $-CH_3$ | $2-CF_3$ | H | H | $-C_6H_5$ | 1 | 0 | 22 (Diast. 1) | 0,47 |
| 10 | $-CH_3$ | $2-CF_3$ | H | H | $-C_6H_5$ | 1 | 0 | 19 (Diast. 2) | 0,45 |

Beispiel 11

5-[2-(2-Hydroxybenzoylamino)-ethoxycarbonyl]-2,6-dimethyl-4-(2-trifluormethylphenyl)-1,4-dihydropyridin-3-carbonsäuremethylester

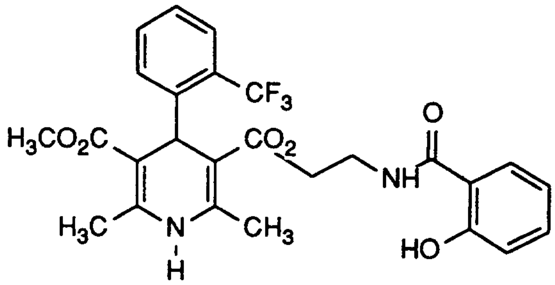

3,4 g (8,5 mmol) 5-(2-Aminomethoxycarbonyl)-2,6-dimethyl-4-(2-trifluormethylphenyl)-1,4-dihydropyridin-3-carbonsäuremethylester, 1,3 g (9,6 mmol) N-Hydroxybenzotriazol und 1,98 g (9,6 mmol) Dicyclohexylcarbodiimid werden in 60 ml abs. THF 1 h bei 0 ° C, dann 2 h bei RT gerührt. Es wird abfiltriert, eingedampft und der Rückstand in Essigsäureethylester aufgenommen. Die Essigesterphase wird mit 2 n HCl, gesättigter $NaHCO_3$-Lösung und mit Wasser gewaschen, über $Na_2SO_4$ getrocknet und eingedampft. Der Rückstand wird über Kieselgel gereinigt (Eluens: Toluol / Essigsäureethylester 10:1).
Ausbeute: 4,09g (92,9%)
$R_f$ = 0,32 (Toluol/Aceton 4:1)
Die freie Hydroxygruppe der Salicylsäure kann durch 30 minütiges Rühren der Salicylsäurederivate in einem Gemisch aus 10 ml Pyridin und 10 ml Acetanhydrid, dem eine Spatelspitze Dimethylaminopyridin zugesetzt ist, acetyliert werden.

In Analogie zur Vorschrift des Beispiels 11 werden die in Tabelle 2 aufgeführten Verbindungen hergestellt:

Tabelle 2:

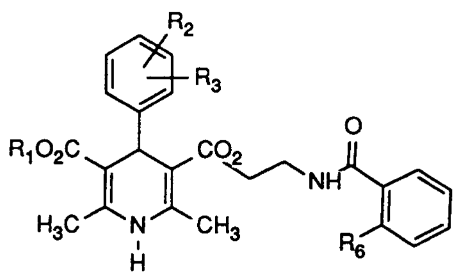

| Bsp.-Nr. | $R^1$ | $R^2$ | $R^3$ | $R^6$ | Ausbeute (% d.Th.) | $R_f$ (Toluol/Aceton 4:1) |
|---|---|---|---|---|---|---|
| 12 | $-CH(CH_3)_2$ | 2-$NO_2$ | 3-H | OH | 82,3 | 0,25 |
| 13 | $-CH_3$ | 2-$CF_3$ | 3-H | -O-CO-$CH_3$ | 79,1 | 0,30 |
| 14 | $-CH_3$ | 2-Cl | 3-Cl | -O-CO-$CH_3$ | 72,7 | 0,31 |
| 15 | $-CH(CH_3)_2$ | 2-$CF_3$ | 3-H | -O-CO-$CH_3$ | 85,2 | 0,33 |
| 16 | $-CH(CH_3)_2$ | 2-$NO_2$ | 3-H | -O-CO-$CH_3$ | 69 | 0,23 |

Beispiel 17

5-[2-(2-Hydroxybenzoyloxy)-2-phenylethoxycarbonyl]-2,5-dimethyl-4-(2-trifluormethylphenyl)-1,4-dihydropyridin-3-carbonsäuremethylester

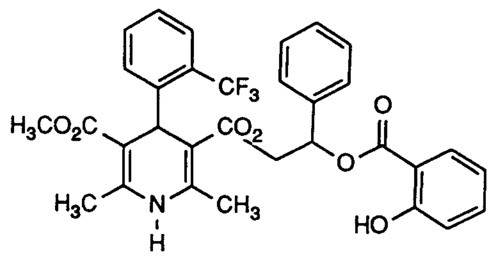

0,637 g (1 mmol) der Verbindung aus Beispiel 9 werden mit 924 mg (1,1 mmol) $NaHCO_3$ in einem Gemisch aus 8 ml MeOH und 2 ml $H_2O$ 2,5 Stunden bei RT gerührt (DC-Kontrolle). Das Methanol wird abgedampft, der Rückstand mit 10 ml eiskalter 1N HCl versetzt und sofort 3 mal mit Essigsäureethylester ausgeschüttelt. Die vereinigten Essigesterphasen werden mit $Na_2SO_4$ getrocknet und eingedampft. Der Rückstand wird durch Chromatographie über Kieselgel mit Toluol/Essigsäureethylester 10:1 als Eluens gereinigt.
Ausbeute: 464 mg (79% d.Th.)
$R_f$ = 0,48 (Toluol/Aceton 4:1)

Beispiel 18

5-[3-(2-Hydroxybenzoyloxy)-2,2-dimethylpropoxycarbonyl]-2,6-dimethyl-4-(2-trifluormethylphenyl)-1,4-dihydropyridin-3-carbonsäuremethylester

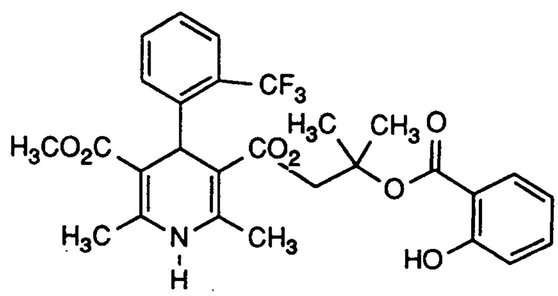

Die Titelverbindung wird in Analogie zur Vorschrift des Beispiels 17 ausgehend von der Acetylverbindung aus Beispiel 8 hergestellt
Ausbeute: 89%
$R_f$ = 0,49 (Toluol/Aceton 4:1)

**Patentansprüche**

**1.** Dihydropyridin-alkoxy- und -alkylamino-aroylester der allgemeinen Formel

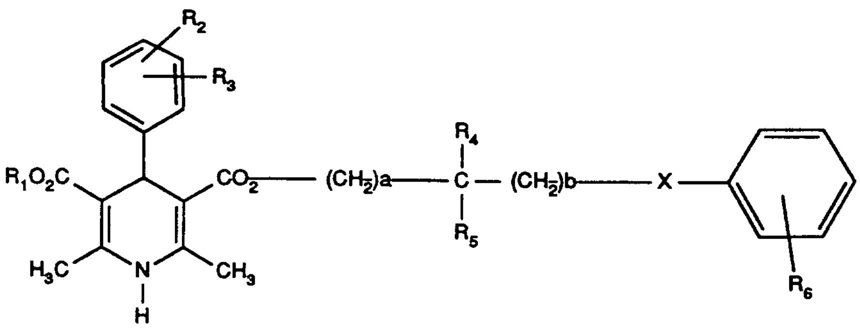 (I),

in welcher

$R^1$ für geradkettiges oder verzweigtes Alkyl mit bis zu 8 Kohlenstoffatomen steht, das gegebenenfalls durch geradkettiges oder verzweigtes Alkoxy mit bis zu 6 Kohlenstoffatomen substituiert ist, oder für Cycloalkyl mit 3 bis 8 Kohlenstoffatomen steht,

$R^2$ für Halogen, Trifluormethyl, Nitro oder Cyano steht,

$R^3$ die oben angegebene Bedeutung von $R^2$ hat und mit dieser gleich oder verschieden ist oder für Wasserstoff steht,

a und b gleich oder verschieden sind und für eine Zahl 0, 1, 2, 3 oder 4 stehen,

$R^4$ und $R^5$ gleich oder verschieden sind und für Wasserstoff, geradkettiges oder verzweigtes Alkyl mit bis zu 6 Kohlenstoffatomen oder Phenyl stehen,

X für eine Gruppe der Formel -O-CO- oder -NH-CO- steht,

$R^6$ für Hydroxy oder für den Rest der Formel

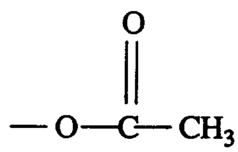

steht,

und deren Salze.

**2.** Dihydropyridin-alkoxy- und -alkylamino-aroylester nach Anspruch 1, wobei

$R^1$ für geradkettiges oder verzweigtes Alkyl mit bis zu 6 Kohlenstoffatomen steht, das gegebenenfalls durch geradkettiges oder verzweigtes Alkoxy mit bis zu 4 Kohlenstoffatomen substituiert ist, oder für Cyclopropyl, Cyclopentyl oder Cyclohexyl steht,

$R^2$ für Fluor, Chlor, Brom, Trifluormethyl, Nitro oder Cyano steht,

$R^3$ die oben angegebene Bedeutung von $R^2$ hat und mit dieser gleich oder verschieden ist, oder für Wasserstoff steht,

a und b gleich oder verschieden sind und für eine Zahl 0, 1, 2 oder 3 stehen,

$R^4$ und $R^5$ gleich oder verschieden sind und für Wasserstoff, geradkettiges oder verzweigtes Alkyl mit bis zu 4 Kohlenstoffatomen oder Phenyl stehen,

X für eine Gruppe der Formel -O-CO-oder -NH-CO- steht,

$R^6$ für Hydroxy oder für den Rest der Formel

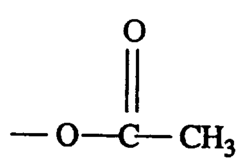

steht,

und deren Salze.

**3.** Dihydropyridin-alkoxy- und -alkylamino-aroylester nach Anspruch 1, wobei

| | |
|---|---|
| $R^1$ | für geradkettiges oder verzweigtes Alkyl mit bis zu 4 Kohlenstoffatomen steht, das gegebenenfalls durch Methoxy substituiert ist, oder für Cyclopentyl oder Cyclohexyl steht, |
| $R^2$ | für Fluor, Chlor, Trifluormethyl, Nitro oder Cyano steht, |
| $R^3$ | die oben angegebene Bedeutung von $R^2$ hat und mit dieser gleich oder verschieden ist oder für Wasserstoff steht, |
| a und b | gleich oder verschieden sind und für eine Zahl 0, 1 oder 2 stehen, |
| $R^4$ und $R^5$ | gleich oder verschieden sind und für Wasserstoff, Methyl oder Phenyl stehen, |
| X | für eine Gruppe der Formel -O-CO oder -NH-CO- steht, |
| $R^6$ | für Hydroxy oder für den Rest der Formel |

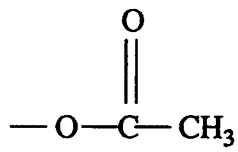

steht,

und deren Salze.

**4.** Dihydropyridin-alkoxy- und -alkylamino-aroylester nach Anspruch 1 aus der Gruppe
5-[2-(2-Acetoxybenzoyloxy)ethoxycarbonyl]-2,6-dimethyl-4-(2-trifluormethylphenyl)-1,4-dihydropyridin-3-carbonsäuremethylester,
5-[2-(2-Acetoxybenzoyloxy)ethoxycarbonyl]-2,6-dimethyl-2-(2-trifluormethylphenyl)-1,4-dihydropyridin-3-carbonsäureethylester,
5-[2-(2-Acetoxybenzoyloxy)ethoxycarbonyl]-2,6-dimethyl-4-(2,3-dichlorphenyl)-1,4-dihydropyridin-3-carbonsäuremethylester.

**5.** Dihydropyridin-alkoxy- und -alkylamino-aroylester nach Anspruch 1 zur therapeutischen Anwendung.

**6.** Verfahren zur Herstellung von Dihydropyridin-alkoxy- und -alkylaminoaroylester nach Anspruch 1, dadurch gekennzeichnet, daß man

[A] im Fall, daß X für die -NH-CO-Gruppe steht, Aminoverbindungen der allgemeinen Formel (II)

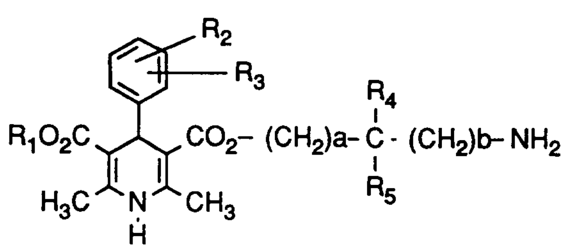

(II),

in welcher

$R^1$, $R^2$, $R^3$, $R^4$, $R^5$, a und b die oben angegebene Bedeutung haben,

mit Salicylsäure der allgemeinen Formel (III)

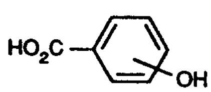

(III)

in inerten Lösemitteln, gegebenenfalls in Anwesenheit einer Base und/oder Hilfsstoffes umsetzt,
im Fall, daß $R^6$ für die $-O-CO-CH_3$-Gruppe steht, mit Acetanhydrid, in Anwesenheit einer Base und eines Hilfsstoffes acetyliert,

[B] und im Fall, daß X für die -O-CO-Gruppe steht, Verbindungen der allgemeinen Formel (IV)

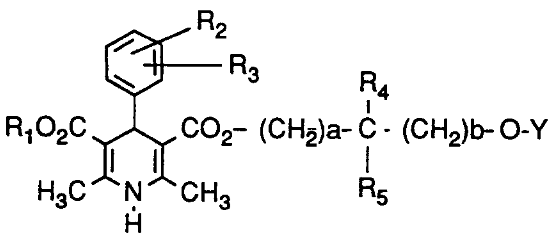

(IV),

in welcher

$R^1$, $R^2$, $R^3$, $R^4$, $R^5$, a und b die oben angegebene Bedeutung haben

und

Y für eine aktivierende Gruppe wie beispielsweise $-SO_3CH_3$, $-SO_3-(C_6H_5)-p-CH_3$ steht,

nach üblichen Methoden einer alkylierenden Veresterung mit Acetylsalicylsäure der Formel (V)

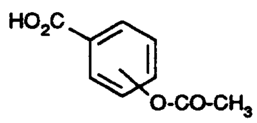

(V)

in inerten Lösemitteln, in Anwesenheit einer Base umsetzt, und im Fall, daß $R^6$ für die Hydroxyschutzgruppe steht, die Acetylgruppe, ebenfalls nach bekannten Methoden, abspaltet.

**7.** Arzneimittel enthaltend mindestens ein Dihydropyridin-alkoxy- und -alkylamino-aroylester nach Anspruch 1.

**8.** Arzneimittel nach Anspruch 7 zur Behandlung von pathologisch verändertem Blutdruck, Herzinsuffizienz, Koronarerkrankungen, thromboembolischen Erkrankungen, Ischämien, transistorische und ischämische Attacken, Angina pectoris, peripheren Durchblutungsstörungen, Arteriosklerose, cerebralen Erkrankungen sowie zur Verhinderung von Okklusion und Restenosen.

**9.** Verfahren zur Herstellung von Arzneimitteln nach Anspruch 7, dadurch gekennzeichnet, daß man die Dihydropyridin-alkoxy- und -alkylamino-aroylester gegebenenfalls mit Hilfe geeigneter Hilfs- und Trägerstoffe in eine geeignete Applikationsform überführt.

**10.** Verwendung von Dihydropyridin-alkoxy- und -alkylamino-aroylester nach Anspruch 1 zur Herstellung von Arzneimitteln.